# EUROPEAN PATENT SPECIFICATION

(11) **EP 3 695 745 B1**
(45) Date of publication and mention of the grant of the patent: **08.09.2021**
(21) Application number: 18867160.6
(22) Date of filing: 23.05.2018
(51) Int. Cl.: A45D 44/00, A61K 8/02, A61Q 1/02, G01J 3/50, G16H 30/40, G16H 20/70

(54) **COSMETIC SHEET AND MANUFACTURING METHOD THEREOF, IMAGE PROCESSING DEVICE, AND IMAGE PROCESSING METHOD**
KOSMETISCHE FOLIE UND HERSTELLUNGSVERFAHREN DAFÜR, BILDVERARBEITUNGSVORRICHTUNG UND BILDVERARBEITUNGSVERFAHREN
FEUILLE COSMÉTIQUE ET SON PROCÉDÉ DE FABRICATION, DISPOSITIF DE TRAITEMENT D'IMAGE, ET PROCÉDÉ DE TRAITEMENT D'IMAGE

(30) Priority: 10.10.2017 JP 2017197068
(43) Date of publication of application: 19.08.2020
(73) Proprietor: Panasonic Intellectual Property Management Co., Ltd., Osaka-shi, Osaka 540-6207 (JP)
(72) Inventor: ONODERA, Mari, Osaka-shi Osaka 540-6207 (JP); SHINODA, Masayo, Osaka-shi Osaka 540-6207 (JP)
(74) Representative: Grünecker Patent- und Rechtsanwälte PartG mbB
(86) International application number: PCT/JP2018/019738
(87) International publication number: WO 2019/073626

(56) References cited:
- EP-A1- 3 216 370
- JP-A- 2000 190 639
- JP-A- 2001 278 739
- JP-A- 2009 100 851
- JP-A- 2010 513 544
- JP-A- 2012 012 339
- JP-A- 2013 136 550
- JP-A- 2013 193 238
- JP-A- 2015 043 836
- JP-A- 2017 518 091

## Description

### Technical Field

The present disclosure relates to a cosmetic sheet and a method for producing the cosmetic sheet, an image-processing apparatus, and an image-processing method.

### Background Art

It has been proposed that a thin film to which an ink containing various coloring materials is applied is put on the human body to obscure a freckle, birthmark, or scar (hereinafter referred to as a "discolored region") on the skin (for example, Patent Literature 1). In a technique described in Patent Literature 1, an image of skin is taken to identify a discolored region. A color similar to the color of the periphery of the discolored region is printed on a thin film, which is put on the skin to obscure the discolored region.

### Citation List

### Patent Literature

PTL 1: Japanese Unexamined Patent Application Publication No. 2015-43836, PL2: EP 3 216 370 A1 (PANASONIC IP MAN CO LTD [JP])

### Summary of Invention

Solution to Problem

In the technique described in Patent Literature 1, however, the color of the periphery of the discolored region is thickly printed to hide the color of the discolored region and to match the periphery. When a cosmetic sheet thus produced is put on the skin, however, the cosmetic sheet tends to arouse a feeling of thickness and contrarily make the discolored portion conspicuous.

One aspect of the present disclosure provides a cosmetic sheet that obscures a discolored portion of the skin and a method for producing the cosmetic sheet. The present disclosure also provides an image-processing apparatus and an image-processing method for producing the sheet. The present invention relates to a cosmetic sheet as defined in claim 1:
A cosmetic sheet to be put on a discolored portion of skin, comprising:a thin film, one surface of which is to be put on the skin;a complementary color layer formed by applying an ink with a color tone complementary to the color of the discolored portion to the other surface of the thin film;a light scattering layer formed by applying an ink containing a reflective material to the complementary color layer; and a coloring layer placed formed by applying a coloring material ink to the light scattering layer,wherein each of the inks for the complementary color layer, the light scattering layer, and the coloring layer further contains a higher alcohol having three to five carbon atoms, purified water, and an acrylic resin,the acrylic resin contains at least one polymer selected from the group consisting of homopolymers of an acrylic monomer and copolymers of two or more acrylic monomers, and wherein the complementary color layer has a basic color of blue and has a maximum at a wavelength in the range of 380 to 570 nm in its spectral reflectance curve.

A cosmetic sheet according to the present disclosure is a cosmetic sheet to be put on a discolored portion of skin, the cosmetic sheet including a thin film, one surface of which is to be put on the skin, a complementary color layer placed on the other surface of the thin film and having a color tone complementary to the color of the discolored portion, a light scattering layer placed on the complementary color layer and containing a reflective material, and a coloring layer placed on the light scattering layer and containing a coloring material.

A cosmetic sheet according to one aspect of the present disclosure put on the skin can obscure a discolored portion of the skin with lower feelings of thickness.

### Brief Description of Drawings

[Fig. 1A] Fig. 1A is an image of a known cosmetic sheet put on the arm.
[Fig. 1B] Fig. 1B is an image of a cosmetic sheet according to the present disclosure put on the arm.
[Fig. 2] Fig. 2 is a spectral reflectance curve of a typical discolored region (reddish freckle), a spectral reflectance curve of a complementary color layer placed on the discolored region, a spectral reflectance curve of a laminate of a light scattering layer and a coloring layer, and a spectral reflectance curve of a skin model.
[Fig. 3A] Fig. 3A is an explanatory view of the reflection of light when no cosmetic sheet is put on.
[Fig. 3B] Fig. 3B is an explanatory view of the reflection of light when a cosmetic sheet is put on.
[Fig. 4A] Fig. 4A is a schematic view of another cosmetic sheet.
[Fig. 4B] Fig. 4B is a schematic view of still another cosmetic sheet.
[Fig. 5] Fig. 5 is a schematic explanatory view of a makeup support system.
[Fig. 6] Fig. 6 is a block diagram of a configuration example of an image-processing apparatus.
[Fig. 7] Fig. 7 is a constitution example of a complementary color layer information table.
[Fig. 8] Fig. 8 is a flow chart of a processing example of the entire image-processing apparatus.
[Fig. 9] Fig. 9 is a flow chart of a processing example of a sheet information providing unit.
[Fig. 10] Fig. 10 is a flow chart of a processing example of a finish checker.
[Fig. 11] Fig. 11 is a schematic view of an example of a finish check screen.
[Fig. 12] Fig. 12 is a block diagram of a hardware configuration example of a computer according to the present disclosure.
[Fig. 13A] Fig. 13A is an image of a cosmetic sheet according to the present disclosure put over a freckle model.
[Fig. 13B] Fig. 13B is an image of a conventional cosmetic sheet according to a comparative example put over the freckle model.

### Description of Embodiments

### 1. Cosmetic Sheet

A cosmetic sheet according to the present disclosure is a sheet to be put on a discolored portion of the skin and, when put on a discolored portion, can obscure the discolored portion or decorate the discolored portion. Examples of a discolored portion of the skin include pigmented spots, chloasmata, nevus spilus, melanocytic nevi, nevus of Ota, acquired dermal melanocytosis, erythema, purpura, leukoderma, bruising, lentigines, blackhead, sunburn regions, acne (pimples), acne scars, pigmentation caused by friction or inflammation, wrinkles, ephelides (freckles), tattoos, verrucae, and scars.

A cosmetic sheet according to the present disclosure may be a sheet for a particular individual (a sheet produced on demand) produced for a discolored portion of the skin of the particular individual, for example, using a makeup support system described later. A cosmetic sheet according to the present disclosure may be a sheet for large numbers of the general public produced for ordinary skin discoloration (for example, reddish discoloration, such as pigmented spots, chloasma, or ephelides, or bluish discoloration, such as purpura or bruising) in large numbers of the general public.

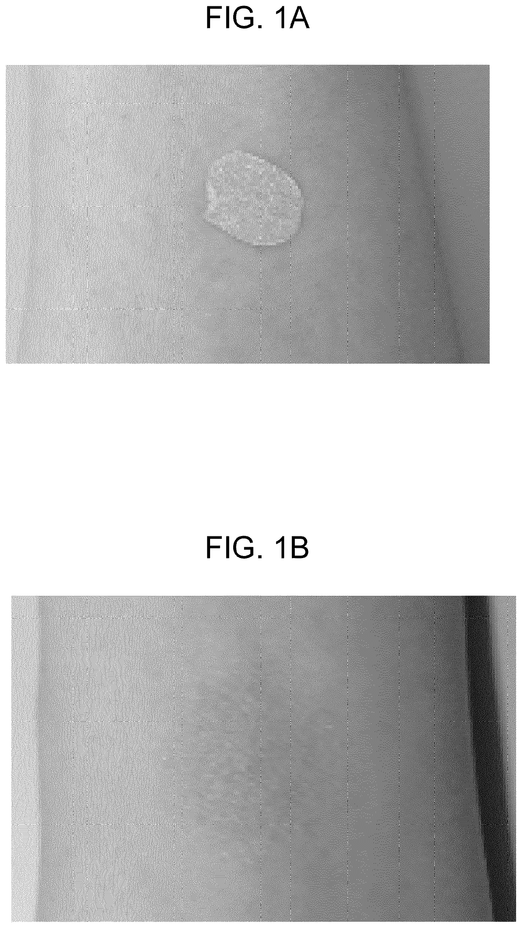

In a known cosmetic sheet, the color tone of the sheet is usually deepened to hide a discolored region in a discolored portion and to obscure the discolored region. A sheet with a deepened color tone, however, has a feeling of thickness and tends to make a region on which the sheet is put conspicuous, for example, as shown in Fig. 1A. Fig. 1A is a photograph of a cosmetic sheet composed of three skin-colored films laminated on a thin film put on the arm. Furthermore, in such a method, even a sheet put on the skin has difficulty in sufficiently hiding a discolored region.

In contrast, a cosmetic sheet according to the present disclosure includes a thin film to be put on the skin, a complementary color layer, a light scattering layer, and a coloring layer laminated in this order. Such a cosmetic sheet can not only obscure a discolored region but also suppress a feeling of thickness, thus obscuring the portion on which the cosmetic sheet is put, as shown in Fig. 1B. Fig. 1B is a photograph of a cosmetic sheet composed of a complementary color layer, a light scattering layer, and a coloring layer laminated on a thin film put on the arm.

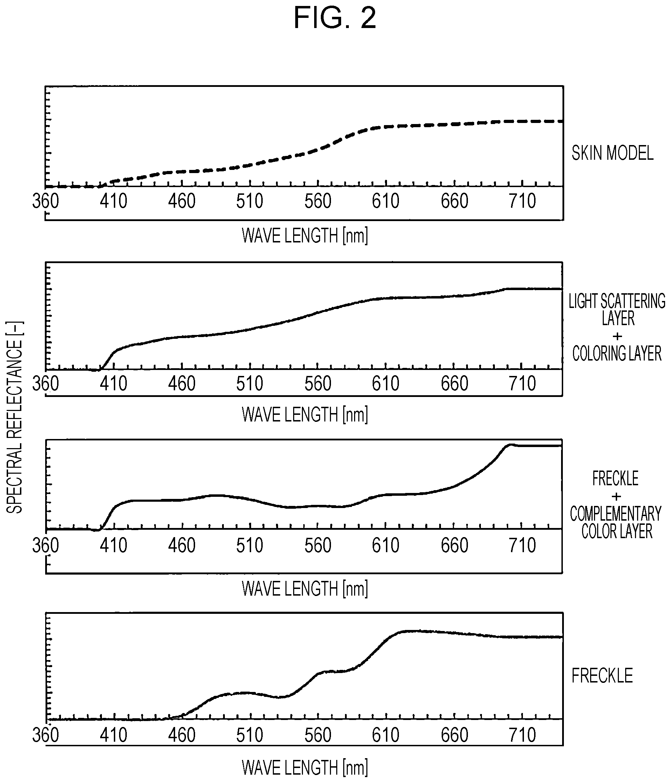

The reason for that is described below with an example in which a cosmetic sheet according to the present disclosure is put on a reddish discolored region. The cosmetic sheet includes a bluish complementary color layer. Fig. 2 shows the spectral reflectance of a freckle (reddish), the spectral reflectance of a complementary color layer (bluish) placed on the freckle, the spectral reflectance of a laminate of a light scattering layer and a coloring layer, and the spectral reflectance of a skin model. As shown in Fig. 2, the spectral reflectance at a wavelength in the range of 410 to 460 nm (a blue region) is lower in the discolored region of the skin than in the skin model. Furthermore, the discolored region of the skin has a region with a higher spectral reflectance than the skin model at a wavelength in the range of 540 to 580 nm (a yellow to red region). A bluish complementary color layer complementary in color to the discolored region put on the discolored region increases the reflectance at a wavelength in the range of 410 to 460 nm. More specifically, this covers a deficiency in a blue color component. On the other hand, this decreases the excessively high reflectance at a wavelength in the range of 540 to 580 nm. Thus, the complementary color layer corrects the region with a large difference between the discolored region and the skin model. The thin film is transparent, and the spectral reflectance of the thin film is ignored. The light scattering layer and the coloring layer placed on the complementary color layer can easily achieve a spectral reflectance curve similar to that of the skin model and can obscure the discolored region even with a small thickness.

In a typical cosmetic sheet, even a skin-colored coloring layer placed on a layer with a relatively deep color tone, such as a complementary color layer, cannot achieve the desired color of the cosmetic sheet because the color of such a complementary color layer can be seen through the skin-colored coloring layer. Thus, it has been difficult to form such a layer. In contrast, a cosmetic sheet according to the present disclosure includes a light scattering layer on a complementary color layer. Thus, it is difficult to visually recognize not only the color of the discolored region but also the color of the complementary color layer.

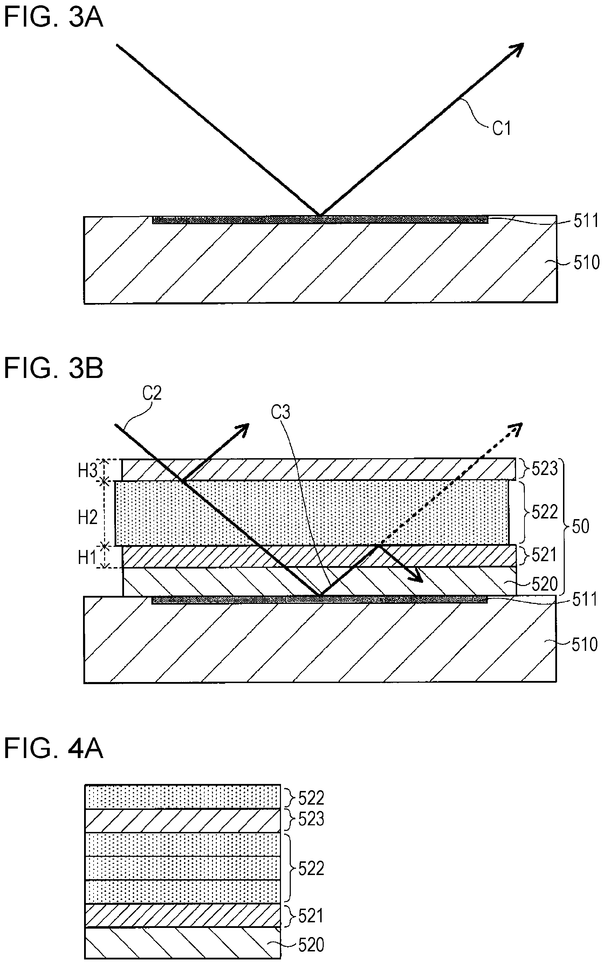

This is more specifically described below. First, as illustrated in Fig. 3A, when a cosmetic sheet 50 is not put on a discolored portion 510, light C1 reflected from a discolored region 511 is directly visually recognized. In contrast, as illustrated in Fig. 3B, when the cosmetic sheet 50 according to the present disclosure (a thin film 520, a complementary color layer 521, a light scattering layer 522, and a coloring layer 523) is put on the discolored portion 510, the color component of reflected light visually recognized is a mixture of color components of the complementary color layer 521, the light scattering layer 522, the coloring layer 523, and the discolored region 511, for example. As described above, the thin film 520 is transparent, and the color of the thin film 520 is ignored.

In the cosmetic sheet 50 according to the present disclosure, the light scattering layer 522 contains a reflective material, which reflects most of incident light C2 on the surface of the light scattering layer 522. Reflected light C3 passing through the light scattering layer 522 and reflected from the discolored region 511 travels toward the front side of the cosmetic sheet 50, during which the reflected light C3 is reflected or scattered by the light scattering layer 522. The reflected light C3 reflected from the discolored region 511 therefore rarely reaches the front side of the cosmetic sheet 50. Likewise, most of light (not shown) reflected from the complementary color layer 520 is reflected or scattered by the light scattering layer 522. Thus, most of such light rarely reaches the front side of the cosmetic sheet 50. Thus, most of visually recognized light is light reflected from the neighborhood of the surface of the coloring layer 523 or the light scattering layer 522, and the color of the complementary color layer 521 or the discolored region 511 is rarely visually recognized. Light reflected from the discolored region 511, passing through the coloring layer 523, and appearing on the front side interferes with reflected light from the complementary color layer 521 and thereby becomes achromatic. Thus, in the cosmetic sheet 50 according to the present disclosure, the color of the discolored region 511 is rarely visually recognized.

The thickness H2 of the light scattering layer 522 may be smaller than but is preferably greater than the thickness H1 of the complementary color layer 521. This enables the reflected light C3 reflected from the discolored region 511 or light reflected from the complementary color layer 521 to be sufficiently reflected from the light scattering layer 522, further obscuring the color of the discolored region 511 or the complementary color layer 521. An excessively large thickness H2 of the light scattering layer 522, however, may result in strong gloss and a feeling of thickness. Thus, the light scattering layer 522 preferably has a thickness in the range of 0.1 to 20, more preferably 0.1 to 10, when the complementary color layer 521 has a thickness of 1. In the case where a plurality of the light scattering layers 522 are laminated, the total thickness of the light scattering layers 522 is preferably in this range. The complementary color layer 521 preferably has a thickness in the range of 0.1 to 6 µm, more preferably 0.1 to 1 µm.

The thickness H3 of the coloring layer 523 depends on the desired color of the cosmetic sheet and preferably ranges from 0.1 to 5, more preferably from 0.1 to 3 when the complementary color layer 521 has a thickness of 1. In the case where a plurality of the coloring layer 523 are laminated, the total thickness of the coloring layer 523 is preferably in this range.

Each layer of the cosmetic sheet is described in detail below.

### 1-1. Thin Film

The thin film 520 is preferably a biocompatible sheet member that does not arouse an uncomfortable feeling when put on the skin of humans. The thin film 520 may be colored, clear and colorless, or translucent, without losing the objects and advantages of the present disclosure.

The thin film 520 preferably has a thickness in the range of 10 nm to 10 µm, more preferably 10 to 1000 nm. In particular, if the thin film 520 is hydrophobic, the thickness particularly preferably ranges from 10 to 800 nm. The thin film 520 may be of any shape, for example, rectangular when viewed from the top. For example, the thin film 520 may also have a shape that matches the shape of the discolored region 511 or its periphery.

The thin film 520 may be formed by a spin coating method, a roll-to-roll method, or a Langmuir-Blodgett (LB) method or may be a fiber sheet composed of folded fibers produced by electrospinning. The thin film 520 may have a peripheral and/or in-plane cut to match the shape of the discolored region 511 or its periphery.

Examples of the material of the thin film 520 include polyesters exemplified by poly(glycolic acid), poly(lactic acid), polycaprolactone, poly(ethylene succinate), poly(ethylene terephthalate), and copolymers thereof; polyethers exemplified by poly(ethylene glycol) and polypropylene glycol); polyamides exemplified by nylon, poly(glutamic acid), poly(aspartic acid), and salts thereof; polysaccharides exemplified by pullulan, cellulose, starch, chitin, chitosan, alginic acid, hyaluronic acid, and corn starch, and salts thereof; silicones exemplified by acrylic silicone and trimethylsiloxysilicic acid; acrylic acids exemplified by alkyl acrylates, silicone acrylate, acrylamide, and copolymers thereof; poly(vinyl alcohol); polyurethane; polycarbonate; poly(acid anhydride); polyethylene; polypropylene; porous layer coating sheets, and nanofiber sheets. The material of the thin film 520 is preferably poly(lactic acid), cellulose (for example, carboxymethylcellulose, hydroxyethylcellulose, etc.), starch, chitin, chitosan, alginic acid, corn starch, or polyurethane, in terms of biocompatibility, availability, and handleability.

### 1-2. Complementary Color Layer

The complementary color layer 521 is placed on the thin film 520 and has a color tone complementary to the color of the discolored region 511 in the discolored portion 510 on which the cosmetic sheet 50 is put. Colors complementary to each other are two opposite colors with respect to the white point on a straight line passing through the white point in the chromaticity diagram. In other words, two different colors that become achromatic (white, gray, or black) when mixed at an appropriate ratio are complementary colors. When a color A of a spectrum is blocked, the color B of the remaining light is complementary to the color A. In the present disclosure, for example, if the color of the discolored region 511 is red to orange, orange to yellow, or brown with lower brightness, the complementary color layer 521 may be a layer with a color tone of green to blue. In the present specification, the "color tone complementary to" the color of the discolored region 511 includes not only the completely complementary color of the discolored region 511 but also similar colors and skin color to which a complementary color is added.

Skin color to which a complementary color is added is peach orange, for example. This is skin color to which blue is added and is effective in covering pigmented spots or dark circles under the eyes. For example, for a discolored region in a yellow range, the complementary color layer 521 may have a bluish color tone that is complementary to yellow, more specifically, light blue, pinkish produced by adding blue to skin color, orangish, a neutral color thereof, that is, peach-orangish, or purplish. For a discolored region in a red range, the complementary color layer 521 may have a greenish color tone that is complementary to red, more specifically, light green. This is effective in covering acne scars. The density depends on the brightness of color of the periphery of the discolored region.

The complementary color layer 521 may be composed of one layer or two or more layers. When the complementary color layer 521 is composed of a plurality of layers, the coloring material in each layer may be of the same type or of different types. The coloring material content of each layer may be the same or different.

The complementary color layer contains at least a coloring material that can reproduce a desired color and, for example, mainly contains the coloring material and a binder. As described later, the complementary color layer 521 may further contain a film forming agent and various additive agents.

Examples of the coloring material in the complementary color layer 521 include inorganic red pigments, such as iron titanate, such as iron oxide and iron hydroxide; inorganic brownish pigments, such as γ-iron oxide; inorganic yellowish pigments, such as yellow iron oxide and ocher; inorganic black pigments, such as black iron oxide and carbon black; inorganic violet pigments, such as manganese violet and cobalt violet; inorganic green pigments, such as chromium hydroxide, chromium oxide, cobalt oxide, and cobalt titanate; inorganic bluish pigments, such as Prussian blue (ferric ferrocyanide), lapis lazuli blue (ultramarine blue), azure, mountain blue, aluminum-cobalt oxide, aluminum-zinc-cobalt oxide, silicon-cobalt oxide, silicon-zinc-cobalt oxide, cobalt pigments, smalt, cobalt blue, cobalt stannate, cobalt chromium blue, cobalt-aluminum-silicon oxide, and manganese blue; organic blue pigments and blue dyes, such as indigo, phthalocyanine, indanthrene blue, and sulfonated products thereof; and various tar dye lakes, various natural dye lakes, and synthetic resin powders produced by combining these powders.

The complementary color layer 521 may contain one coloring material or two or more coloring materials. These coloring materials may be of any shape, for example, acicular, amorphous, spherical, or plate-like.

The median (D50) of the integrated value of the particle size distribution of the coloring material measured by a laser diffraction method preferably ranges from 125 nm to 2 µm, more preferably 125 to 1000 nm. Still more preferably, the average particle size (D50) ranges from 125 to 1000 nm, and the 90% value (D90) of the integrated value of the particle size distribution is 3000 nm or less. When the average particle size (D50) of the particle size distribution of the coloring material is in the above range, the complementary color layer 521 can have a thickness in a desired range, and it is difficult to visually recognize the color of the complementary color layer 521, as described above.

Examples of the binder include particles composed of (meth)acrylic resins, such as alkyl (meth)acrylate polymers, styrene-(meth)acrylate copolymers, alkyl (meth)acrylate-vinyl acetate copolymers, (meth)acrylic acid-alkyl (meth)acrylate copolymers, and alkyl (meth)acrylate dimethicone polymers; vinyl acetate polymers; and vinylpyrrolidone-styrene copolymers. The term "(meth)acryl", as used herein, refers to acryl, methacryl, or a combination of acryl and methacryl.

Among these, the binder is preferably composed of particles of a (meth)acrylic resin (hereinafter also referred to simply as "acrylic particles"). If the binder is composed of acrylic particles, the coloring material tends to have high fixability, and the complementary color layer 521 tends to have high durability. The binder is more preferably composed of a (meth)acrylic resin that does not irritate the skin. Thus, the acrylic particles are preferably selected from components described in the list of cosmetic ingredient label names in the Pharmaceutical Affairs Law in Japan, components in accordance with EU cosmetics regulation (Cosmetics Directive 76/768/EEC), and components described in International Cosmetic Ingredient Dictionary and Handbook (January 1, 2002, 9th edition) of the Cosmetic, Toiletry & Fragrance Association (CTFA) in U.S.A. and are preferably acrylic resin particles used in known cosmetics.

Specific examples of (meth)acrylic resins constituting the acrylic particles include homopolymers of (meth)acrylic monomers, copolymers of two or more (meth)acrylic monomers, and copolymers of (meth)acrylic monomers and other monomers.

Examples of the (meth)acrylic monomers include acrylic acid, methyl acrylate, ethyl acrylate, acrylamide, n-propyl acrylate, n-butyl acrylate, isobutyl acrylate, octyl acrylate, 2-ethylhexyl acrylate, N,N-dimethylaminoethyl acrylate, acrylonitrile, methacrylic acid, ethyl methacrylate, methacrylamide, n-propyl methacrylate, n-butyl methacrylate, isobutyl methacrylate, 2-ethylhexyl methacrylate, octyl methacrylate, hydroxyethyl methacrylate, and N,N-dimethylaminoethyl methacrylate.

Examples of other monomers that can be copolymerized with the (meth)acrylic monomers include styrene, vinyl acetate, silicone macromers, fluorinated monomers, and alkoxy silane unsaturated monomers.

The binder preferably has an average particle size in the range of 30 to 150 nm. The average particle size is the median (D50) of the integrated value of the particle size distribution measured by a laser diffraction method. More preferably, the average particle size (D50) ranges from 30 to 150 nm, and the 90% value (D90) of the integrated value of the particle size distribution is 250 nm or less. When the binder has an average particle size in this range, the coloring material can be easily bonded to the thin film.

The binder content of the complementary color layer 521 preferably ranges from 0.5 to 10 parts by mass, more preferably 1.5 to 5.7 parts by mass, per 10 parts by mass of the coloring material. When the coloring material and binder contents are in this range, the coloring material has high fixability. When the binder content is in this range, the coloring material content relatively tends to become sufficient, and the complementary color layer 521 can have a desired color.

### 1-3. Light Scattering Layer

The light scattering layer 522 is placed on the complementary color layer 521, contains at least a reflective material, and may contain a reflective material and a binder. The light scattering layer 522 may also contain a film forming agent and various additive agents, as described later. As described above, the light scattering layer 522 scatters or reflects light and thereby makes it difficult to visually recognize the color of the discolored region 511 or the complementary color layer 521.

The light scattering layer 522 may be composed of one layer or two or more layers. When the light scattering layer 522 is composed of a plurality of layers, the reflective material in each layer may be of the same type or of different types. The reflective material content of each layer may be the same or different.

The reflective material is composed of particles that scatter or reflect ultraviolet light and visible light (for example, light with a wavelength in the range of 200 to 780 nm), for example, a white pigment, a pearl agent, a soft focus agent, or a lame agent. The pearl agent, the soft focus agent, and the lame agent preferably do not irritate the skin. Examples of these agents include white pigments, such as titanium oxide, zinc oxide, cerium oxide, and barium sulfate; white extender powders, such as talc, muscovite, phlogopite, lepidolite, biotite, synthetic mica, sericite, synthetic sericite, kaolin, silicon carbide, bentonite, smectite, silicic anhydride, aluminum oxide, magnesium oxide, zirconium oxide, antimony oxide, diatomaceous earth, aluminum silicate, aluminum magnesium metasilicate, calcium silicate, barium silicate, magnesium silicate, calcium carbonate, magnesium carbonate, hydroxyapatite, and boron nitride; photoluminescent powders, such as calcium aluminum borosilicate, titanium dioxide coated mica, titanium dioxide coated glass powders, titanium dioxide coated bismuth oxychloride, titanium dioxide coated mica, titanium dioxide coated talc, iron oxide coated mica, iron oxide coated mica titanium, iron oxide coated glass powders, Prussian blue treated mica titanium, carmine treated mica titanium, bismuth oxychloride, argentine, poly(ethylene terephthalate)-aluminum-epoxy layered powders, and poly(ethylene terephthalate)-polyolefin laminated film powders; organic low-molecular-weight powders, such as N-acyl lysine; natural organic powders, such as silk powders and cellulose powders; metal powders, such as aluminum powders, gold powders, and silver powders; composite powders, such as fine titanium oxide coated mica titanium, fine zinc oxide coated mica titanium, barium sulfate coated mica titanium, titanium-oxide-containing silicon dioxide, and zinc-oxide-containing silicon dioxide; kapok fibers; poly(methyl methacrylate) crosspolymers; and non-cross-linked acrylic particles.

The light scattering layer 522 may contain one reflective material or two or more reflective materials. Among these, preferred are titanium oxide, zinc oxide, cerium oxide, aluminum oxide, and magnesium oxide, and particularly preferred are titanium oxide, zinc oxide, and cerium oxide, because these make it difficult to visually recognize the color of the discolored region 511 or the complementary color layer 521.

The reflective material may be of any shape, for example, spherical, plate-like, or acicular. The reflective material preferably has an average particle size in the range of 125 nm to 2 µm. An average particle size of 125 nm or more makes it difficult to visually recognize the color of the discolored region 511 or the complementary color layer 521. The average particle size is the median (D50) of the integrated value of the particle size distribution measured by a laser diffraction method. The reflective material more preferably has an average particle size (D50) in the range of 125 to 1000 nm. Still more preferably, the average particle size (D50) ranges from 125 to 1000 nm, and the 90% value (D90) of the integrated value of the particle size distribution is 3000 nm or less.

The binder in the light scattering layer 522 may be the same as the binder in the complementary color layer 521. The binder content preferably ranges from 0.5 to 10 parts by mass, more preferably 1.5 to 5.7 parts by mass, per 10 parts by mass of the reflective material. When the reflective material and binder contents are in this range, the reflective material has high fixability. When the binder content is in this range, the reflective material content relatively tends to become sufficient, and the light scattering layer 522 can sufficiently reflect or scatter light.

### 1-4. Coloring Layer

The coloring layer 523 is a layer containing a coloring material placed on the light scattering layer 522, for example, a layer containing a coloring material and a binder. The coloring layer 523 may also contain a film forming agent and various additive agents, as described later. The coloring layer 523 adds skin color or a desired color to the discolored region 511 after color correction with the complementary color layer 521 and thereby produces normal skin color or a desired color when observed from the outer surface of the cosmetic sheet 50. As described above, the coloring layer 523 is placed on the complementary color layer 521 and the light scattering layer 522. In a cosmetic sheet according to the present disclosure, the complementary color layer 521 and the light scattering layer 522 (particularly the complementary color layer) make reflected light from the discolored region 511 achromatic. Thus, in the cosmetic sheet 50 according to the present disclosure, the underlying color of the coloring layer 523 has low turbidity, and the coloring layer 523 develops a very good color.

Although the coloring layer 523 usually preferably has a skin color tone, the coloring layer 523 may be of any color when the cosmetic sheet 50 is used as a cosmetic, such as a cheek, eye shadow, or body painting. In the cosmetic sheet 50, although the coloring layer 523 may be entirely of the same color, or the cosmetic sheet 50 may be entirely of uniform color, the coloring layer 523 may partly have a different color region.

The coloring layer 523 may be composed of one layer or two or more layers. In the coloring layer 523 composed of a plurality of layers, the coloring material in each layer may be of the same type or of different types. The coloring material content of each layer may be the same or different. For example, a coloring layer of any color may be laminated on a skin-colored coloring layer.

The coloring layer 523 with a skin color tone may have any color. For example, the spectral reflectance curve of the coloring layer measured at a wavelength in the range of 400 to 700 nm may have a first inflection point at a wavelength in the range of 550 to 610 nm and a second inflection point at a wavelength of less than 550 nm. In such a case, preferably, the spectral reflectance at the first inflection point is higher than the spectral reflectance at the second inflection point, and the spectral reflectance at a wavelength of more than 580 nm is equal to or higher than the spectral reflectance at a wavelength of 580 nm. The coloring layer 523 with such a spectral reflectance has a small difference in color from the skin when the cosmetic sheet 50 is put on the skin (for example, "Mongoloid skin"), and the region on which the cosmetic sheet 50 is put tends to become inconspicuous. The spectral reflectance of the coloring layer 523 may be measured with a spectrophotometer (for example, CM-700d manufactured by Konica Minolta, Inc.) using the main light source: D50 and the measurement mode: SCE. The term "inflection point", as used herein, refers to a point at which the positive and negative of curvature in the curved line are reversed.

When the coloring layer 523 has the above spectral reflectance curve, the spectral reflectance at a wavelength in the range of 590 to 700 nm is preferably 1 to 1.3 times, more preferably 1 to 1.2 times, higher than the spectral reflectance at a wavelength of 580 nm. When the spectral reflectance of the coloring layer 523 at a wavelength in the range of 590 to 700 nm is within the above range, the color of the coloring layer 523 is closer to the color of the skin of humans. The coloring layer 523 has brightness (L* value in the L*a*b* color system (CIE 1976)) in the range of 48 to 80, more preferably 48 to 73. The coloring layer 523 with brightness in this range tends to have a color closer to the color of the skin of humans. The brightness may also be measured with a spectrophotometer (for example, CM-700d manufactured by Konica Minolta, Inc.) using the main light source: D50 and the measurement mode: SCE.

The color (spectral reflectance) or brightness of the coloring layer 523 can be adjusted by a combination of coloring materials. For example, white, red, and yellow coloring materials in combination with a blue or black coloring material can more easily achieve the above spectral reflectance or brightness.

The binder in the coloring layer 523 may be the same as the binder in the complementary color layer 521. The binder content of the coloring layer 523 preferably ranges from 0.5 to 10 parts by mass, more preferably 1.5 to 5.7 parts by mass, per 10 parts by mass of the coloring material. When the coloring material and binder contents are in this range, the coloring material has high fixability. When the binder content is in this range, the coloring material content relatively tends to become sufficient, and the coloring layer 523 can have a desired color.

### 1-5. Other Constituents

The cosmetic sheet 50 may include a layer other than the complementary color layer 521, the light scattering layer 522, and the coloring layer 523 without losing the objects and advantages of the present disclosure. For example, a soft focus layer that contains the above reflective material, such as a soft focus agent, a lame agent, or a pearl agent, to show finer skin by light scattering may be disposed on the coloring layer 523. A hygroscopic layer that contains a moisture absorbent to control the humidity on the surface of the cosmetic sheet and to increase the comfort of the cosmetic sheet may be disposed on the coloring layer 523. Examples of the moisture absorbent include spherical silica, porous acrylic particles, and nylon 6 (polyamide 6).

Although the thin film 520, the complementary color layer 521, the light scattering layer 522, and the reflective layer 523 are laminated in this order in the cosmetic sheet 50 described above, the cosmetic sheet 50 may further include a reflective layer 522 on the coloring layer 523, for example, as illustrated in Fig. 4A. In this case, the uppermost reflective layer 522 can scatter the color of the coloring layer 523 and further obscure the cosmetic sheet 50 when put on the skin.

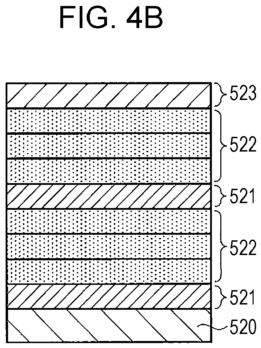

For example, as illustrated in Fig. 4B, the coloring layer 523 may be placed on the complementary color layers 521 and the light scattering layers 522 alternately laminated.

### 1-6. Method for Producing Cosmetic Sheet

The cosmetic sheet can be produced by performing a step of applying a complementary color layer ink containing a coloring material complementary to the color of a discolored portion of the skin to one surface of a thin film (the thin film described above), the other surface of which is to be put on the skin, and drying the complementary color layer ink to form a complementary color layer, a step of applying a light scattering layer ink containing a reflective material to the complementary color layer and drying the light scattering layer ink to form a light scattering layer, and a step of applying a coloring layer ink containing a coloring material to the light scattering layer and drying the coloring layer ink to form a coloring layer.

The complementary color layer ink, the light scattering layer ink, and the coloring layer ink (hereinafter also collectively referred to as the cosmetic ink) may be applied by any method, including a known method. Examples of the application method include an ink jet printing method, a screen printing method, offset printing, and gravure printing. Among these, the ink jet method is preferred in terms of the practicability of on-demand printing or laminated printing, in which the cosmetic ink is applied multiple times. A method for producing a cosmetic sheet is described in the following example in which the cosmetic ink is applied by the ink jet method. However, the present disclosure is not limited to this method.

The cosmetic ink can be applied by the ink jet method using any ink jet apparatus, for example, of a known piezoelectric system, thermal system, or electrostatic system. Among these, an ink jet apparatus of a piezoelectric device system is preferred because it obviates the need for heating, which is required in a thermal ink jet system.

The cosmetic ink may be applied one or two or more times in each step. When the cosmetic ink is applied multiple times in each step, the cosmetic ink may be dried each time or after applied multiple times.

The cosmetic ink may be dried by any method that can remove the solvent (for example, a higher alcohol described later or purified water) from the cosmetic ink. For example, the cosmetic ink may be dried at atmospheric pressure and at room temperature or may be dried by heating to a predetermined temperature and/or by reducing the pressure. For heating, heating to a temperature in the range of, for example, 25°C to 50°C is preferred. In such a range, the cosmetic ink can be efficiently dried without deterioration of the thin film or the solid component of the cosmetic ink. For reducing the pressure, the pressure is preferably reduced by -0.1 to 0 MPa. Under such a reduced pressure, the cosmetic ink can be efficiently dried.

The complementary color layer ink applied in the step of forming the complementary color layer may be any ink containing a coloring material, for example, an ink containing the above coloring material, the above binder, a higher alcohol, and purified water. If necessary, the ink may contain a film forming agent and various additive agents.

Likewise, the light scattering layer ink applied in the step of forming the light scattering layer may be any ink containing a reflective material, for example, an ink containing the above reflective material, the above binder, a higher alcohol, and purified water. If necessary, the ink may contain a film forming agent and various additive agents.

The coloring layer ink applied in the step of forming the coloring layer may be any ink containing a coloring material, for example, an ink containing the above coloring material, the above binder, a higher alcohol, and purified water. If necessary, the ink may contain a film forming agent and various additive agents.

Although the complementary color layer ink, the light scattering layer ink, and the coloring layer ink may be different inks and may be individually applied in each step, the complementary color layer ink, the light scattering layer ink, and the coloring layer ink may be prepared as common inks, which are applied in each step. More specifically, the ink tanks of the ink jet apparatus may be filled with white ink, red ink, yellow ink, blue ink, black ink, and optionally an ink containing a reflective material. To form each layer, these inks may be appropriately combined to form desired layers. In such a case, the white ink may also be used as a light scattering layer ink.

A higher alcohol, purified water, a film forming agent, and various additive agents in the cosmetic ink are described below.

### • Higher Alcohol

The higher alcohol may be any higher alcohol that has three or more carbon atoms and that is compatible with purified water. The higher alcohol functions as a solvent for the cosmetic ink. After the cosmetic ink is applied, the higher alcohol is absorbed into the thin film or volatilizes.

The higher alcohol preferably has 3 to 5, more preferably 3 or 4, carbon atoms. The higher alcohol having such a number of carbon atoms is easily compatible with purified water.

The higher alcohol preferably contains a trivalent alcohol. The higher alcohol containing a trivalent alcohol potentially prevents the higher alcohol or purified water from volatilizing excessively within various printers. Consequently, the cosmetic ink can be stably applied with the printers. In this case, the cosmetic ink has a constant viscosity and can stably form desired images.

The trivalent alcohol may be any trivalent alcohol that does not irritate the skin and is preferably glycerin. Glycerin is biologically safe. Furthermore, glycerin in the cosmetic ink tends to suppress aggregation of a coloring material or a reflective material and potentially prevents an increase in viscosity of the cosmetic ink during long-term storage.

The higher alcohol may contain a divalent alcohol or a monovalent alcohol. Examples of the divalent alcohol include diethylene glycol, propylene glycol, 1,3-propanediol, butylene glycol, and hexanediol. Examples of the monovalent alcohol include propanol, isopropanol, and butyl alcohol. Among these, a divalent alcohol, particularly propylene glycol, is preferred. Divalent alcohols have lower viscosity than purified water and trivalent alcohols and have low surface tension. Thus, a divalent alcohol in the cosmetic ink improves the wettability of the cosmetic ink to the thin film and potentially prevents unevenness of an image produced.

The total higher alcohol content is preferably 20 parts or less by mass, more preferably 10 to 20 parts by mass, per 100 parts by mass of the cosmetic ink. An excessively high higher alcohol content tends to result in aggregation of a reflective material or a coloring material. On the other hand, 20 parts or less by mass of the higher alcohol potentially prevents aggregation of a reflective material or a coloring material and enables the cosmetic ink to be stably ejected from various printers.

The amount of trivalent alcohol per 100 parts by mass of the cosmetic ink is determined according to the method of applying the cosmetic ink. For example, for the cosmetic ink to be applied with an ink jet apparatus, the amount of trivalent alcohol is preferably 20 parts or less by mass, more preferably 10 to 20 parts by mass. When the amount of trivalent alcohol is in such a range, a higher alcohol or purified water in the cosmetic ink has appropriately controlled volatility, and the cosmetic ink can be stably ejected from an ink jet apparatus.

The amount of divalent alcohol per 100 parts by mass of the cosmetic ink is also determined according to the method of applying the cosmetic ink. For example, for the cosmetic ink to be applied with an ink jet apparatus, the amount of divalent alcohol is preferably 20 parts or less by mass, more preferably 10 to 20 parts by mass. The cosmetic ink containing such an amount of divalent alcohol can easily have a viscosity in a desired range.

### • Purified Water

Purified water also functions as a solvent for the cosmetic ink. After the cosmetic ink is applied, purified water is absorbed into the thin film or volatilizes.

The purified water may be any purified water generally used for cosmetics and may be water purified by various methods such as distillation and ion exchange. For example, the purified water may be hot spring water, deep water, or steam-distilled water of plants.

The amount of purified water is preferably 10 parts or more by mass, more preferably 20 parts or more by mass, per 100 parts by mass of the cosmetic ink.

### • Film Forming Agent

The film forming agent is a compound that improves the film formation (for example, drying characteristics) of the cosmetic ink. The "film forming agent", as used herein, refers to a compound that can be dispersed in water at room temperature (except the component corresponding to the binder). The cosmetic ink may contain one or two or more film forming agents.

The film forming agent may be a compound that can be dispersed or dissolved in a higher alcohol and/or purified water, for example, at least one compound selected from the group consisting of acrylic polymers, polysaccharide polymers, sugar alcohols, sterols, esters, and modified corn starches. The cosmetic ink may contain one or two or more film forming agents. The film forming agent composed of a compound selected from the group can greatly accelerate the drying of a coating film formed of the cosmetic ink.

The film forming agent preferably has an HLB value of 8 or more, more preferably 8 to 19. A film forming agent with an HLB value of 8 or more can be easily uniformly dispersed or dissolved in a higher alcohol or purified water. The HLB value is an index representing the relative affinity ratio for oil and water in an oil-water system. In general, a substance having a higher HLB value has a higher affinity for water. The HLB value in the present specification is determined by the Griffin method.

The film forming agent is also preferably a material that does not irritate the skin. Examples of the acrylic polymers include alkyl acrylate copolymers, 2-amino-2-methyl-1-propanol salts (hereinafter also referred to as "AMP") of alkyl acrylate copolymers, sodium salts (hereinafter also referred to as "Na") of alkyl acrylate copolymers, alkyl acrylate copolymer ammonium, acrylic acid-alkyl acrylate copolymers, alkyl acrylate-diacetone acrylamide copolymers, alkyl acrylate-diacetone acrylamide copolymer AMPs, 2-amino-2-methyl-1,3-propanediol salts (hereinafter also referred to as "AMPD") of alkyl acrylate-diacetone acrylamide copolymers, hydroxyethyl acrylate-methoxyethyl acrylate copolymers, hydroxyethyl acrylate-butyl acrylate-methoxyethyl acrylate copolymers, acrylate-alkyl acrylate (1 to 18 carbon atoms)-alkyl (1 to 8 carbon atoms) acrylamide copolymer AMPs, alkyl acrylate-octylacrylamide copolymers, acrylate-t-butylacrylamide copolymers, acrylate-ethylhexyl acrylate copolymers, acrylate copolymers, acrylate copolymer AMPs, acrylate copolymer Na, polyurethane-14-acrylate copolymer AMP, vinyl acetate-butyl maleate-isobornyl acrylate copolymers, styrene-alkyl acrylate copolymers, styrene-acrylate copolymers, styrene-acrylamide copolymers, polyurethane-1 (a compound represented by INCI name: POLYURETHANE-1), polyacrylate-22 (a compound represented by INCI name: POLYACRYLATE-22), tricontanyl polyvinylpyrrolidone (PVP), (eicosene/vinylpyrrolidone) copolymers, and (vinylpyrrolidone/hexadecene) copolymers.

Examples of the polysaccharide polymers include gum arabic, glucan, succinoglycan, carrageenan, karaya gum, tragacanth gum, guar gum, locust bean gum, galactomannan gum, xanthan gum, starch, carob gum, quince seed (Cydonia oblonga), casein, dextrin, gelatin, sodium pectate, sodium alaginate, methylcellulose, ethylcellulose, carboxymethylcellulose, hydroxyethylcellulose, hydroxypropylcellulose, crystalline cellulose, O-[2-hydroxy-3-(trimethylammonio)propyl]hydroxyethylcellulose chloride, O-[2-hydroxy-3-(trimethylammonio)propyl]guar gum chloride, O-[2-hydroxy-3-(trimethylammonio)propyl]locust bean gum chloride, starch hydroxypropyltrimonium chloride, glyceryl glucoside, glycosyl trehalose, Tremella polysaccharide, and dextrin isostearate.

Examples of the sugar alcohols include sorbitol, maltitol, and glucose. The sterols are compounds with a sterol skeleton. Examples of the sterols include phytosterols, such as campesterol, campestanol, brassicasterol, 22-dehydrocampesterol, stigmasterol, stigmastanol, 22-dihydrospinasterol, 22-dehydrostigmastanol, 7-dehydrostigmasterol, sitosterol, tirucallol, euphol, fucosterol, isofucosterol, codisterol, clionasterol, poriferasterol, clerosterol, 22-dehydroclerosterol, fungisterol, chondrillasterol, avenasterol, vernosterol, and pollinastanol; zoosterols, such as cholesterol, dihydrocholesterol, cholestanol, coprostanol, epicoprosterol, epicoprostanol, 22-dehydrocholesterol, desmosterol, 24-methylenecholesterol, lanosterol, 24,25-dihydrolanosterol, norlanosterol, spinasterol, dihydroagnosterol, agnosterol, lophenol, and lathosterol; mycosterols, such as dehydroergosterol, 22,23-dihydroergosterol, episterol, ascosterol, and fecosterol; and hydrogenated compounds thereof.

Examples of the esters include dipentaerythritol fatty acid esters, such as hexa(hydroxystearic acid/stearic acid/rosin acid) dipentaerythrityl, (hydroxystearic acid/stearic acid/rosin acid) dipentaerythrityl, hexahydroxystearic acid dipentaerythrityl, tetra(hydroxystearic acid/isostearic acid) dipentaerythrityl, (hydroxystearic acid/isostearic acid) dipentaerythrityl; hydrogenated castor oil fatty acid esters, such as stearic acid hydrogenated castor oil, isostearic acid hydrogenated castor oil, and hydroxystearic acid hydrogenated castor oil; cholesterol fatty acid esters, such as hydroxystearic acid cholesteryl; phytosterol fatty acid esters, such as phytosteryl oleate and macadamia nut oil fatty acid phytosteryl; hydrogenated vegetable oils, such as hydrogenated coconut oil and hydrogenated palm oil; dimer dilinoleic acid (phytosteryl/isosteryl/cetyl/stearyl/behenyl); pentahydroxystearic acid sucrose; and lauroyl glutamic acid di(octyldodecyl/phytosteryl/behenyl).

The modified corn starches may be corn starches modified with any compound without losing the objects and advantages of the present disclosure and may be hydroxypropyl modified starches produced by a reaction between corn starch and 3-(dodecenyl)dihydro 2,5-furandione.

Among these, preferred in terms of improved drying characteristics of the cosmetic ink are acrylate copolymers, acrylate (ethylhexyl acrylate) copolymers, polyurethane-14-acrylate copolymer AMPs, alkyl acrylate copolymer ammonium, dimer dilinoleyl bis(behenyl/isostearyl/phytosteryl) dimer dilinoleate-hydrogenated triglyceryl rosinate, xanthan gum crosspolymer-hydroxyethylcellulose, Tremella polysaccharide, modified corn starches, and dextrin isostearate, and more preferred are acrylate copolymers, acrylate (ethylhexyl acrylate) copolymers, polyurethane-14-acrylate copolymer AMPs, alkyl acrylate copolymer ammonium, dimer dilinoleyl bis(behenyl/isostearyl/phytosteryl) dimer dilinoleate-hydrogenated triglyceryl rosinate, xanthan gum crosspolymer-hydroxyethylcellulose, and Tremella polysaccharide.

The amount of film forming agent is preferably 20 parts or less by mass, more preferably 0.3 to 5 parts by mass, per 100 parts by mass of the cosmetic ink. As described above, 0.3 parts or more by mass of the film forming agent can improve the drying characteristics of the cosmetic ink. Although an excessively large amount of the film forming agent may result in the cosmetic ink with excessively high viscosity, 20 parts or less by mass of the film forming agent can result in the cosmetic ink with a viscosity suitable for various printing methods.

In the preparation of the cosmetic ink, the film forming agent is typically dissolved in a solvent and is mixed with a coloring material, a reflective material, a binder, a higher alcohol, and purified water. The solvent is also preferably a solvent that does not irritate the skin, such as the above higher alcohol or water.

The cosmetic ink may further contain a water-soluble polymer (a polymer not corresponding to the film forming agent) that binds the reflective material or the coloring material to a substrate, without losing the objects and advantages of the present disclosure.

### • Various Additive Agents

Various additive agents are also preferably compounds with negative skin irritancy. Examples of the various additive agents include surfactants, pH-adjusting agents, thickeners, ultraviolet absorbers, ultraviolet scattering agents, antiseptic antimold agents, deoxidizers, antioxidants, antiseptic agents, antifading agents, antifoaming agents, perfumes, and solvents other than higher alcohols and purified water.

### • Physical Properties of Cosmetic Ink

The cosmetic ink preferably has a viscosity of 50 mPa·s or less, more preferably 1 to 20 mPa·s, still more preferably 3.5 to 8 mPa·s, measured with a cone-plate viscometer at a rate of 100 (1/s) at 25°C. The cosmetic ink with a viscosity in such a range can be easily applied with various printers. In particular, the cosmetic ink with a viscosity in such a range can be easily and stably ejected from an ink jet apparatus.

The cosmetic ink preferably has a pH in the range of 6 to 10, more preferably 7.5 to 9.5. The cosmetic ink with a pH in such a range does not erode members of various printers, and the coloring material rarely aggregates during long-term storage of the cosmetic ink. Thus, a film of a desired color can be easily formed.

The cosmetic ink preferably has a surface tension of 50 mN/m or less, more preferably 32 to 46 mN/m, at 25°C. The cosmetic ink with a surface tension of 50 mN/m or less applied to various substrates with various printers has good wettability and can form a film with a uniform thickness. Although the surface tension can be measured by various methods, the above values are measured by the pendant drop method, which is employed in general-purpose apparatuses.

The average particle size of the particles in the cosmetic ink, that is, the median (D50) of the integrated value of the particle size distribution measured by a laser diffraction method preferably ranges from 125 nm to 2 µm. More preferably, the average particle size (D50) ranges from 125 to 1000 nm. Still more preferably, the average particle size (D50) ranges from 125 to 1000 nm, and the 90% value (D90) of the integrated value of the particle size distribution is 3000 nm or less. The cosmetic ink containing particles with an average particle size in such a range can be stably ejected from various printers, particularly ink jet apparatuses. The cosmetic ink with D50 or D90 in such a range can be stably ejected from an ink jet apparatus.

Preferably, the cosmetic ink has negative skin irritancy, that is, is biologically safe. The cosmetic ink with negative skin irritancy can be used in applications involving contact with the skin. The term "negative skin irritancy", as used herein, refers to cell viability of more than 50% in a test using a three-dimensional skin model, which is an alternative method to the skin irritancy test. In the alternative method to the skin irritancy test, a 5% sodium dodecyl sulfate (SDS) solution is used as an irritancy control, and phosphate-buffered saline (PBS) is used as a negative control. After the ink is exposed to the three-dimensional skin model for 18 hours, cell viability is determined in an MTT test.

In one method of providing the cosmetic ink with negative skin irritancy, all the components in the cosmetic ink are selected from components described in the list of cosmetic ingredient label names in the Pharmaceutical Affairs Law in Japan, components in accordance with EU cosmetics regulation (Cosmetics Directive 76/768/EEC), and components described in International Cosmetic Ingredient Dictionary and Handbook (January 1, 2002, 9th edition) of the Cosmetic, Toiletry & Fragrance Association (CTFA) in U.S.A.

### • Method for Preparing Cosmetic Ink

The cosmetic ink is produced by mixing a coloring material, a reflective material, a binder, a higher alcohol, purified water, and another optional component in a dispersing apparatus. The components can be mixed in a dispersing apparatus, such as a known ball mill, sand mill, rolling mill, homomixer, or attritor.

### 2. Makeup Support System for On-Demand Production of Cosmetic Sheet

As described above, the cosmetic sheet may be produced for a discolored portion of the skin of a particular individual. A makeup support system for producing such an individual cosmetic sheet is described below.

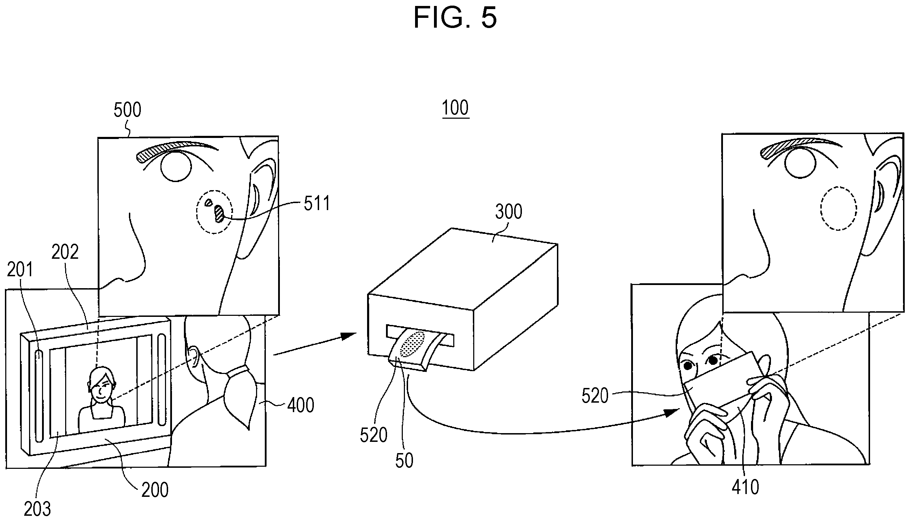

Fig. 5 illustrates an example of a makeup support system 100. The makeup support system 100 includes an image-processing apparatus 200 including an illumination unit 201, a camera 202, and a display screen 203, such as a touch screen liquid crystal display, and a printer 300 communicatively coupled to the image-processing apparatus 200.

In the image-processing apparatus 200, the face of a user 400 in front of the display screen 203 irradiated with visible light emitted from the illumination unit 201 is photographed with the camera 202 located near the display screen 203. The image-processing apparatus 200 horizontally reverses the photographed image to produce a face image 500, which is displayed on the display screen 203. Thus, the user 400 feels as if the user looks at itself in the mirror.

The image-processing apparatus 200 identifies at least one discolored region 511 from the face image 500 (or the face image before reversal). The image-processing apparatus 200 then provides print data for producing a cosmetic sheet 50 that obscures the discolored portion of the skin 410 corresponding to the identified discolored region 511 in the face image 500. The image-processing apparatus 200 then sends the print data to the printer 300 coupled to the image-processing apparatus 200 via a specified network or cable.

When the user 400 uses the cosmetic sheet 50 not only to obscure the discolored portion 511 but also as a cosmetic, such as a cheek, eye shadow, or tattoo, the image-processing apparatus 200 may provide print data for producing the cosmetic sheet 50 on demand from the user 400. In this case, the image-processing apparatus 200 provides print data according to the individual size or position on the basis of the feature point information of the individual analyzed by an image analyzer 230.

When the image-processing apparatus 200 provides such print data for the cosmetic sheet 50, the display screen 203 with a touch panel may display the face image of the user 400 together with makeup parts, and the user 400 can select colors using a color picker function or a color pallet or select a desired design, size, or position.

The printer 300 may combine various inks contained in its ink tanks according to the print data sent from the image-processing apparatus 200 and form a complementary color layer, a light scattering layer, and a coloring layer in a desired pattern on a thin film (laminated printing), thereby producing the sheet 50.

The user 400 can put the cosmetic sheet 50 on a discolored portion of the skin 410 to obscure the discolored portion. When the image-processing apparatus 200 identifies no discolored region 511, the printer 300 may form a sheet without the complementary color layer 521 and the light scattering layer 522, that is, the sheet 50 that includes the coloring layer 523 on the thin film 520 and that matches the size of the individual skeleton. The display screen 203 may indicate that there is no discolored portion 510, and the user 400 may decide to print nothing or to print the coloring layer 523 corresponding to a foundation.

The image-processing apparatus 200 and the printer 300 are placed in a factory, a cosmetics store, a beauty salon, a medical institution, a makeup room for personal appearances, an event site, or a private house, for example. The image-processing apparatus 200 may be a portable apparatus that can be easily carried. The image-processing apparatus 200 is described below.

### • Image-Processing Apparatus

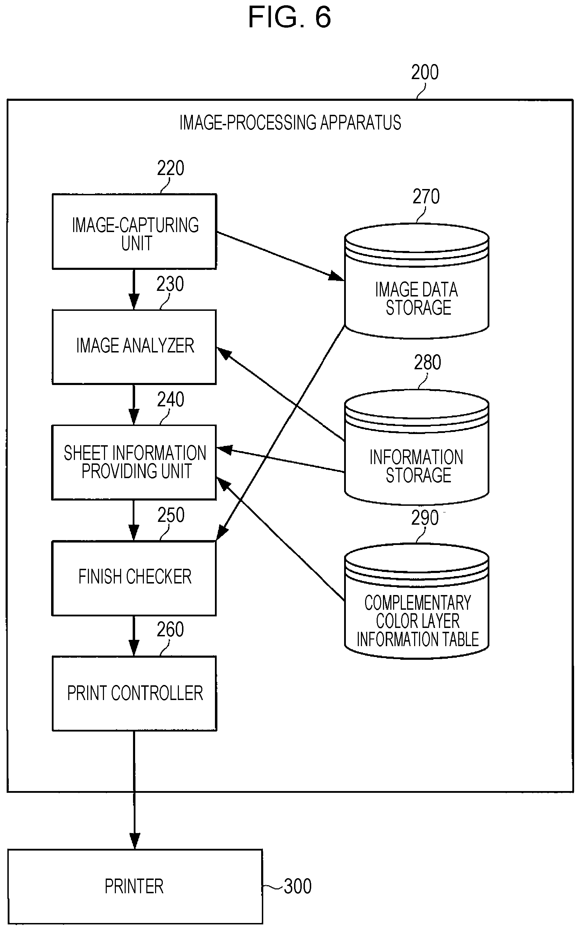

Fig. 6 illustrates an example of the configuration of the image-processing apparatus 200. For example, the image-processing apparatus 200 includes an image-capturing unit 220, the image analyzer 230, a sheet information providing unit 240, a finish checker 250, a print controller 260, an image data storage 270, an information storage 280, and a complementary color layer information table 290.

The image data storage 270 stores image data acquired by the image-capturing unit 220.

The information storage 280 stores in advance various pieces of information necessary for image analysis with the image analyzer 230 and various pieces of information necessary to determine sheet information with the sheet information providing unit 240.

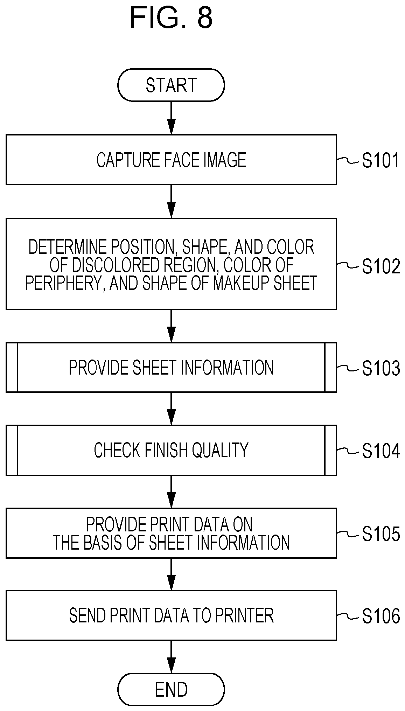

The complementary color layer information table 290 manages complementary color layer information that matches the color and the like of the discolored region 511. The complementary color layer information determines the structure of the complementary color layer 521 or the light scattering layer 522 of the cosmetic sheet 50. The complementary color layer information table 290 is described in detail later (see Fig. 8).

The image-capturing unit 220 captures the face image 500 of the user 400 taken with the camera 202 (see Fig. 5) and stores the face image 500 in the image data storage 270. The image-capturing unit 220 outputs the captured face image 500 to the image analyzer 230.

The image analyzer 230 analyzes the face image 500, identifies one or two or more discolored regions 511, and determines the shape and color of the discolored regions 511. The image analyzer 230 determines the color of the periphery of the discolored region(s) 511. The image analyzer 230 determines the shape of the cosmetic sheet 50 on the basis of the shape of the discolored region(s) 511. The processing in the image analyzer 230 is described in detail later.

The sheet information providing unit 240 determines, from the complementary color layer information table 290, the complementary color layer 521 and the light scattering layer 522 that match the color and the like of the discolored region(s) 511 determined by the image analyzer 230. The sheet information providing unit 240 also determines a matched coloring layer 523 on the basis of the color of the periphery of the discolored region(s) 511 determined by the image analyzer 230. The sheet information providing unit 240 then provides sheet information containing information about the determined color, thickness, and the like of the complementary color layer 521, the light scattering layer 522, and the coloring layer 523. The processing in the sheet information providing unit 240 is described in detail later.

The finish checker 250 produces and displays an image of the cosmetic sheet 50 formed according to the sheet information and put on the skin, allowing the user to check the finish quality of the cosmetic sheet 50. The finish checker 250 receives instructions to adjust the color of the cosmetic sheet 50 from the user 400. The finish checker 250 is described in detail later.

The print controller 260 provides print data for producing the cosmetic sheet 50 with the printer 300 on the basis of the sheet information provided by the sheet information providing unit 240 and adjusted by the finish checker 250. The print controller 260 sends the print data to the printer 300 via a specified network, cable, or the like.

The printer 300 performs laminated printing of the complementary color layer 521, the light scattering layer 522, and the coloring layer 523 on the thin film 520 according to the print data sent from the print controller 260. Thus, the cosmetic sheet 50 is formed.

The printer 300 may include the print controller 260. In such a case, the image-processing apparatus 200 sends sheet information to the printer 300 via a network or cable, and the print controller 260 in the printer 300 provides print data from the received sheet information. In this case, the sheet information is provided with identification information for identifying the user 400.

### <Details of Complementary Color Layer Information Table>

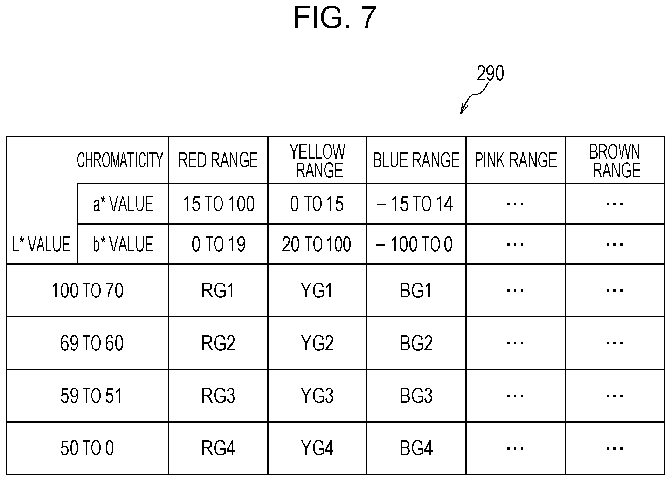

Referring to Fig. 7, the complementary color layer information table 290 is described in detail below.

The complementary color layer information table 290 defines the relationship between the CIE 1976 (L*, a*, b*) color space (hereinafter referred to as the "L*a*b* color space") and the structure of the complementary color layer.

More specifically, as shown in Fig. 7, the complementary color layer information table 290 defines grades in which the brightness (L* value) is divided by specified ranges and the color ranges in which the chromaticity (a* and b* values) is divided by specified ranges.

In the example shown in Fig. 7, the brightness (L* value) is divided into Grade 1 "100 to 70", Grade 2 "69 to 60", Grade 3 "59 to 51", and Grade 4 "50 to 0", and the chromaticity is divided into a red range with an a* value of "15 to 100" and a b* value of "0 to 19", a yellow range with an a* value of "0 to 15" and a b* value of "20 to 100", and a blue range with an a* value of "-15 to 14" and a b* value of "-100 to 0". The color ranges may include a pink range and a brown range in addition to the red range, yellow range, and blue range. The threshold of the brightness (L* value) may be altered in each color range and may be divided into five or more.

The complementary color layer information table 290 assigns a piece of complementary color layer information to a combination of grade and color range. Complementary color layer information to which a combination of grade and color range is assigned contains the structure information of the complementary color layer and the light scattering layer that obscure the discolored portion 510 as much as possible when the discolored region 511 has brightness of this grade and chromaticity in this color range. The structure information about the complementary color layer 521 may contain information about the thickness and color of the complementary color layer 521, and the structure information about the light scattering layer 522 may contain information about the thickness of the light scattering layer 522. The information about thickness may be expressed in length, such as in nanometer, or in the number of laminated prints.

Thus, the image-processing apparatus 200 identifies, from the complementary color layer information table 290, the complementary color layer information to which the brightness (L* value) and chromaticity (a* and b* values) of the discolored region 511 are assigned, thereby enabling the formation of the cosmetic sheet 50 including the complementary color layer 521 and the light scattering layer 522 that further obscure the discolored portion.

The thresholds of the a* and b* values to divide chromaticity in Fig. 7 are only examples and may be altered depending on the type of the camera 202, a polarizing filter, and/or the illumination environment. The chromaticity may be divided into a yellow range of (b* value) ≥ 1.284 x (a* value) and a red range of (b* value) < 1.284 x (a* value), for example.

### <Total Processing in Image-Processing Apparatus>

Referring to the flow chart of Fig. 8, processing in the image-processing apparatus 200 is described below.

First, the image-capturing unit 220 captures the face image 500 taken with the camera 202, stores the face image 500 in the image data storage 270, and outputs the face image 500 to the image analyzer 230 (S101).

The image analyzer 230 then analyzes the face image 500 and determines the position, shape, and color of the discolored region 511, the color of the periphery of the discolored region 511, and the shape of the cosmetic sheet 50 (S102). The processing in the image analyzer 230 is described in detail later.

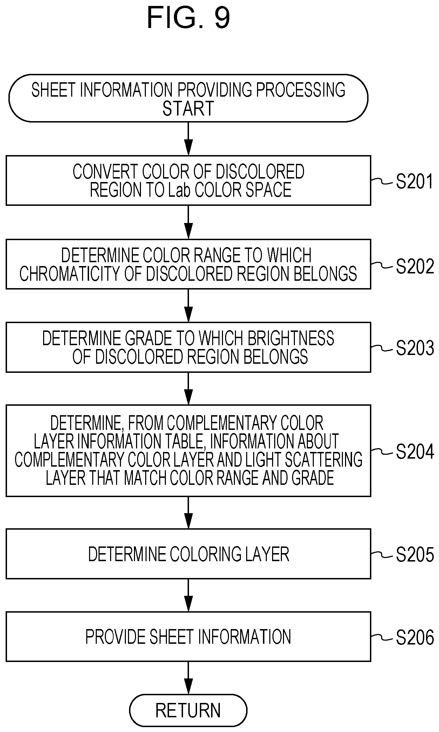

The sheet information providing unit 240 then provides sheet information (S103). The processing of the sheet information providing unit 240 is described in detail later (see Fig. 9).

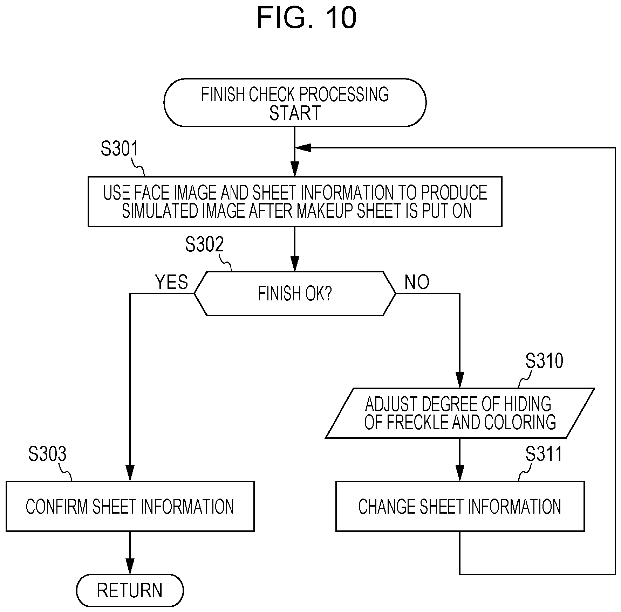

The finish checker 250 then allows the user 400 to check the finish quality of the cosmetic sheet 50 formed according to the sheet information and put on the skin and receives adjustment of sheet information from the user 400 (S104). The processing of the finish checker 250 is described in detail later (see Fig. 10).

The print controller 260 provides print data on the basis of the sheet information (S105).

Finally, the print controller 260 sends the print data to the printer 300 (S106).

Receiving the print data, the printer 300 performs laminated printing of the complementary color layer 521, the light scattering layer 522, and the coloring layer 523 on the thin film 520 according to the print data to form the cosmetic sheet 50.

### <Details of Processing in Image Analyzer>

The processing in the image analyzer 230 in the step S102 is described in detail below.

First, the image analyzer 230 captures an image of a specified color chart and determines the correction value of color correction depending on the photographing environment on the basis of the color of the color chart in the image.

The image analyzer 230 then performs color correction with the determined correction value and detects the position of a face component from the face image 500 through known image recognition processing, such as pattern matching.

The image analyzer 230 then identifies as a skin region a region of a predetermined color range in the region of the face image 500 except the face component.

The image analyzer 230 then divides the skin region into a discolored region and a non-discolored region by a specified pixel value (for example, brightness) threshold to identify the discolored region 511. The image analyzer 230 may treat a discolored region with no more than a specified area as a non-discolored region.

The image analyzer 230 then calculates the average pixel value of a non-discolored region near the discolored region 511 to determine the color of the periphery of the discolored region 511. The color of the periphery of the discolored region 511 may be determined on the basis of the values measured with an external measuring instrument, such as a spectrophotometer, or may be determined by selection from a color sample prepared in advance. Alternatively, the color of the periphery of the discolored region 511 may be determined from the color information of a foundation, concealer, or the like used by the user. This is expected to improve color consistency with a cosmetic used in combination with the cosmetic sheet 50.

The image analyzer 230 then determines the shape of the cosmetic sheet 50 from the arrangement of the face component. For example, the image analyzer 230 determines, as the shape of the cosmetic sheet 50, a closed shape that can cover one or two or more identified discolored regions 511 (or a region with a specified width outside the discolored regions 511) except the positions of face components (nostrils, eyes, mouth, eyebrows, etc.). Because the face is three-dimensional, and the cosmetic sheet 50 is basically flat, it is desirable that the shape of the cosmetic sheet 50 be limited to 5 cm or less x 5cm or less.

Through the above processing, the image analyzer 230 determines the position, shape, and color of the discolored region(s) 511 and the color of the periphery of the discolored region(s) 511 in the face image 500, and the shape of the cosmetic sheet 50.

The image analyzer 230 may identify different discolored regions in the discolored region 511. For example, the image analyzer 230 identifies color in each pixel unit (pixel) in the discolored region 511. For example, this enables the production of the cosmetic sheet 50 that can appropriately obscure a discolored portion containing a deep-colored portion within a light-colored portion.

The image analyzer 230 may convert the color information of the discolored region 511 based on the CMYK or RGB color model to color information based on the L*a*b* color space (hereinafter referred to as "Lab color information").

When pixels with the grade of the minimum L* value (the darkest grade) constitute at least a specified proportion of the discolored region 511, the image analyzer 230 may employ the grade of the minimum L* value as the overall grade of the discolored region 511. This enables the production of the cosmetic sheet 50 that does not necessarily precisely adjust its angle and position to the discolored portion when put on the discolored portion. In other words, the cosmetic sheet 50 can be produced that is convenient to the user. The "specified proportion" may be controlled as a variable parameter by the information storage 280.

When the discolored region 511 includes a plurality of regions with different color ranges and with the same grade, the image analyzer 230 may employ the color range of the largest region as the overall color range of the discolored region 511.

The image analyzer 230 may identify the class of the discolored portion by the color and/or shape of the discolored region 511.

### <Details of Processing in Sheet Information Providing Unit>

Referring to the flow chart of Fig. 9, processing in the sheet information providing unit 240 in the step S103 is described in detail below.

First, the sheet information providing unit 240 converts the color information of the discolored region 511 based on the CMYK or RGB color model identified by the image analyzer 230 to Lab color information (S201). Likewise, the sheet information providing unit 240 also converts the color of the periphery of the discolored region 511 to Lab color information. The conversion processing to Lab color information may be performed by the image analyzer 230 instead of the sheet information providing unit 240.

The sheet information providing unit 240 then determines the color range in the complementary color layer information table 290 to which the chromaticity (a* and b* values) in the Lab color information of the discolored region 511 belongs (S202).

The sheet information providing unit 240 then determines the grade in the complementary color layer information table 290 to which the brightness (L* value) in the Lab color information of the discolored region 511 belongs (S203).

The sheet information providing unit 240 then determines, from the complementary color layer information table 290, complementary color layer information that matches the color range determined in the step S202 and the grade determined in the step S203 (S204). This determines the structure (color, thickness, etc.) of the complementary color layer 521 and the light scattering layer 522.

The sheet information providing unit 240 then determines the structure of the coloring layer 523 on the basis of the Lab color information of the periphery of the discolored region 511 (S205). For example, the sheet information providing unit 240 determines, as the color of the coloring layer 523, the color that most closely matches the Lab color information of the periphery of the discolored region 511. The color of the coloring layer 523 is not necessarily determined on the basis of the Lab color information of the periphery of the discolored region 511 and may be determined on the basis of Lab color information instructed in advance by the user, for example.

The color of the coloring layer 523 may also be determined by the following procedure. For example, the Lab color information of the periphery of the discolored region 511 is stored in a mesh space in which each of the L* a*, and b* axes is graduated in intervals of 3 in the L*a*b* color space. The intervals (specified partition width) are determined depending on the decrease in the difference between a sheet color and the skin color of the periphery of the sheet, may be determined arbitrarily, and may be other than 3. Likewise, the candidate color database applied to the complementary color layer 521, the light scattering layer 522, and the coloring layer 523 is stored in a similar mesh space. The candidate color database covers all the colors of any color representation and is considered to be one database applied to the three layers. The candidate color database, however, is not limited to this, and databases (three in this example) corresponding to each layer may be configured to have a color tone group applied to each layer.

Furthermore, the sheet information providing unit 240 holds a color database A viewed from the top of the complementary color layer 521, the light scattering layer 522, and the coloring layer 523 laminated in this order, the complementary color layer 521 being adjacent to the discolored portion, with respect to all combinations thereof and stores the color database A in a similar mesh space. The database A also holds information about combinations of the complementary color layer 521, the light scattering layer 522, and the coloring layer 523.

Referring to coordinates including a color combination of the complementary color layer 521 determined from the Lab color information of the discolored region 511 in the database A, the coordinates are compared with the coordinates of the Lab color information of the periphery of the discolored region 511 on the mesh to select the closest coordinates. The resulting combination of the complementary color layer 521, the light scattering layer 522, and the coloring layer 523 is applied to the structure of the sheet.

Finally, the sheet information providing unit 240 provides sheet information including the structure of the complementary color layer 521 and the light scattering layer 522 determined in the step S204, the structure of the coloring layer 523 determined in the step S205, the position and shape of the discolored region 511, the Lab color information of the discolored region 511, the Lab color information of the periphery of the discolored region 511, and information about the shape of the cosmetic sheet 50 (S206).

Through these processing steps, the sheet information providing unit 240 provides sheet information for producing the cosmetic sheet 50.

When the image analyzer 230 identifies the class of the discolored region 511, the sheet information providing unit 240 may adjust the structure of the complementary color layer 521, the light scattering layer 522, and/or the coloring layer 523 for the identified class of the discolored region 511.

For example, in the case where peach orange is applied to the complementary color layer 521 in a user with a discolored region of the yellow range, brightness (L*) of the color of the periphery of the discolored region higher than a threshold requires a decrease in the density of the peach orange in the complementary color layer 521. In the case of strong blueness in the color of the periphery of a discolored region, to improve complexion, pink may be added to an upper portion of the periphery of the discolored region (that is, a layer adjacent to the complementary color color 521 on the top of the discolored region). This can cover the discolored region and also make the entire skin including the periphery of the discolored region look healthy.

In an example to treat a darkening of the skin in a discolored portion, to obscure the darkening and impart transparency using a sheet, blue is selected as the color of the complementary color layer 521. When acquired brightness (L*) of the color of the periphery of a discolored region in a user with darkening is higher than a threshold, purple is applied to the complementary color layer 521. In this case, to form (print) a first functional layer, purple may be applied to the entire surface of the first functional layer, or blue or pink dots or lines may be distributed on the surface.

Furthermore, to adjust the structure of the complementary color layer 521, the light scattering layer 522, and/or the coloring layer 523, the light scattering layer 522 and/or the coloring layer 523 may have the same hue and the same brightness in the thickness direction. The determined color of the complementary color layer 521 may have a different shade of color in the thickness direction. For example, a gradual change from a deep color to a light color may occur from the complementary color layer 521 to the coloring layer 523 in the thickness direction.

### <Details of Processing in Finish Checker>

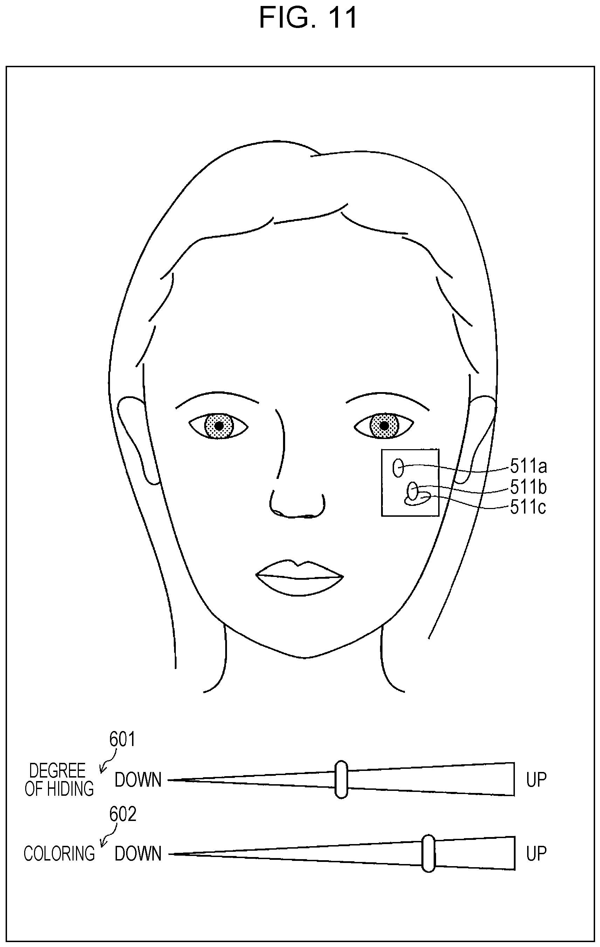

Referring to the flow chart of Fig. 10 and the finish check screen of Fig. 11, processing of the finish checker 250 in the step S104 is described in detail below.

First, the finish checker 250 uses the face image 500 captured in the step S101 and the sheet information provided in the step S105 to produce a simulated image, in which the cosmetic sheet 50 formed according to the sheet information is put on the face of the user 400 (S301).

The finish checker 250 then displays the simulated image produced in S301 on the display screen 203 and inquires of the user 400 whether the user 400 accepts the finish quality of the cosmetic sheet 50 (S302).

If the user 400 accepts the finish quality of the cosmetic sheet 50 (S302: YES), then the finish checker 250 confirms the sheet information (S303) and completes the processing.

If the user 400 does not accept the finish quality of the cosmetic sheet 50 (S302: NO), as illustrated in Fig. 11, the finish checker 250 displays a user interface (Ul) 601 for adjusting the degree of hiding of the discolored portion and a UI 602 for adjusting the coloring of the cosmetic sheet 50 and receives adjustment from the user 400 (S310). In the presence of a plurality of discolored regions 511, the finish checker 250 may display Uls 511a, 511b, and 511c for selecting the discolored region(s) 511 to be adjusted by the user 400.

The user 400 operates these Uls to adjust the color of the cosmetic sheet 50. For example, the user 400 operates the UI 602 for adjusting coloring to adjust the coloring of the cosmetic sheet 50 so as to further harmonize with the color of the periphery or so as to match the color of a foundation usually used by the user 400. The user 400 operates the UI 601 for adjusting the degree of hiding of the discolored portion to adjust the brightness of the cosmetic sheet 50.

The adjustment performed by the user in the step S310 is then reflected in the sheet information by the finish checker 250 (S311). Typically, the adjustment of coloring by the UI 602 is reflected in the color of the coloring layer 523, and the adjustment of the degree of hiding of the discolored portion by the UI 601 is reflected in the color of the complementary color layer 521 or the thickness of the light scattering layer 522. The finish checker 250 then returns to the step S301 and produces another simulated image in which the cosmetic sheet 50 formed according to the adjusted sheet information is put on.

Through these processing steps, the finish checker 250 allows the user 400 to check the finish quality of the cosmetic sheet 50 before the step of printing the cosmetic sheet 50. If necessary, the finish checker 250 allows the user 400 to adjust the finish quality of the cosmetic sheet 50.

The finish checker 250 is not necessarily an essential constituent in the image-processing apparatus 200. There may be a printing configuration without the user checking the finish or a printing mode without the user's knowledge.

### • Advantages of the Image-Processing Apparatus

The image-processing apparatus 200 identifies, from a face image of a discolored portion, the discolored region 511 corresponding to the discolored portion, determines the complementary color on the basis of the a* and b* values of the L*a*b* color space in the identified discolored region 511, and determines the color of the complementary color layer 521. The thickness of the light scattering layer 522 is determined on the basis of the L*, a*, and b* values of the L*a*b* color space in the discolored region 511 and/or the color of the complementary color layer 521. Thus, the cosmetic sheet 50 that can further obscure the discolored portion can be produced.

### <Hardware Configuration>

The image-processing apparatus 200 according to the present disclosure is described in detail above with reference to the drawings. The function of the image-processing apparatus 200 can be performed using a computer program.

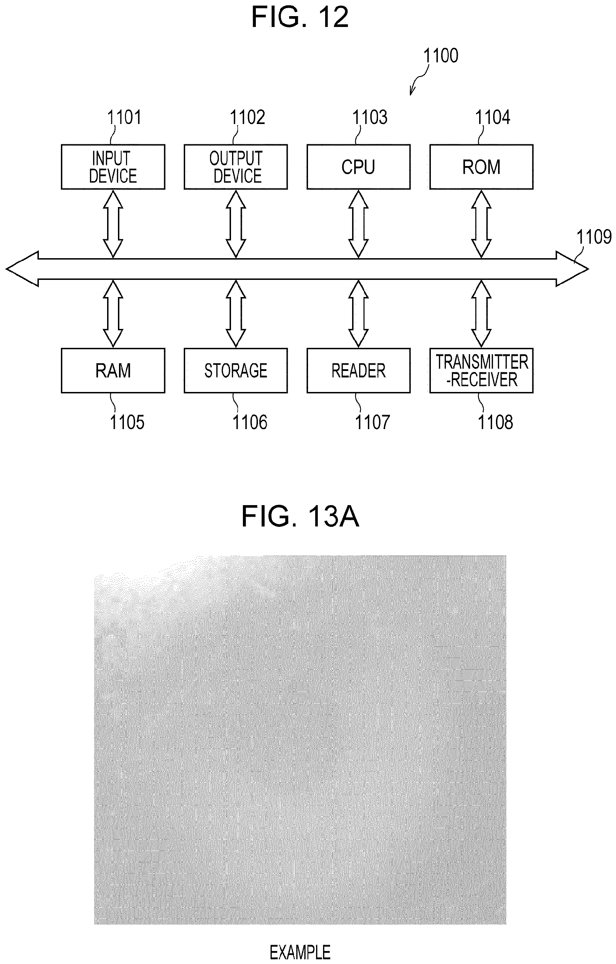

Fig. 12 illustrates the hardware configuration of a computer that performs the function of the apparatus 200 using a program. A computer 1100 includes an input device 1101, such as a keyboard, a mouse, or a touchpad, an output device 1102, such as a display or a speaker, a central processing unit (CPU) 1103, a read only memory (ROM) 1104, a random access memory (RAM) 1105, a storage 1106, such as a hard disk drive or a solid state drive (SSD), a reader 1107, such as a digital versatile disk read only memory (DVD-ROM) or a universal serial bus (USB) memory, for reading information from a recording medium, and a transmitter-receiver 1108 for communication via a network. These components are coupled to one another via a bus 1109.

The reader 1107 reads a program for performing the function of the apparatus 200 from a recording medium that records the program, and stores the program in the storage 1106. Alternatively, the transmitter-receiver 1108 communicates with a server coupled to a network and stores a program for performing the function of each apparatus or device downloaded from the server in the storage 1106.

The CPU 1103 copies the program stored in the storage 1106 into the RAM 1105, successively reads instructions contained in the program from the RAM 1105, and runs the instructions to perform the function of the apparatus 200.

### EXAMPLES

The present disclosure is described in the following example. The present disclosure is not limited to the example.

### 1. Preparation of Cosmetic Ink

### 1-1. Materials

The following materials were used in the example. The average particle size of particles is the median (D50) of the integrated value of the particle size distribution measured by a laser diffraction method.

### (A) Coloring Materials

Reddish coloring material: inorganic reddish pigment (average particle size: 150 nm)
Yellowish coloring material: inorganic yellowish pigment (average particle size: 150 nm)
Bluish coloring material: inorganic bluish pigment (average particle size: 150 nm)
Blackish coloring material: inorganic blackish pigment (average particle size: 150 nm)
Whitish coloring material: inorganic whitish pigment (reflective material) (average particle size: 950 nm)

### (B) Higher Alcohols

Glycerin
1,3-propanediol

### (C) Purified water

### (D) Binder

Acrylic polymer particles (average particle size: 50 nm)

### 1-2. Preparation of Cosmetic Ink

The materials were mixed at the component ratio listed in Table 1 below to prepare a cosmetic ink. Table 1 also lists the physical properties of each cosmetic ink. In this example, the white ink also acts as a light scattering layer ink.

**[Table 1]**

| | | White ink | Red ink | Yellow ink | Blue ink | Black ink |
|---|---|---|---|---|---|---|
| (A) Coloring mterials | Reddish coloring material (mass%) | | 10-15 | | | |
| | Yellowish coloring material (mass%) | | | 10-15 | | |
| | Bluish coloring material (mass%) | | | | 10-15 | |
| | Blackish coloring material (mass%) | | | | | 10-15 |
| | Whitish coloring material (mass%) | 10-15 | | | | |
| (B) Higher Alcohols | Glycerin (mass%)/1,3-propanediol (mass%) | 10/10 | | | | |
| (C) Purified water (mass%) | | 62.5-67.5 | | | | |
| (D) Binder | Acrylic polymer particles (mass%) | 2.5 | | | | |
| Viscosity (mPa·s) | | 5.5 | 5.5 | 5.5 | 5.5 | 5.5 |
| pH | | 8.0 | 8.0 | 8.0 | 8.0 | 8.0 |
| Surface tension (mN/m) | | 34 | 34 | 34 | 34 | 34 |

### 2. Production of Cosmetic Sheet

### 2-1. Example

The white ink, red ink, yellow ink, blue ink, and black ink were charged into ink tanks of an ink jet apparatus equipped with an LB3 ink jet head manufactured by Panasonic Precision Device. A poly(lactic acid) sheet 200 nm in thickness was put on a filter paper support to prepare a substrate.

A circular colored paper sheet that imitates a freckle was placed on a standard skin model (Bioskin (manufactured by Beaulax Co., Ltd., product number BIO)) to prepare a freckle model. The L* a*, and b* values of the L*a*b* color space of a region corresponding to the freckle on the freckle model were measured with a spectrophotometer (CM-700d manufactured by Konica Minolta, Inc.) using the main light source: D50 and the measurement mode: SCE. The color tone of the complementary color of the region (blue (color with a maximum at a wavelength in the range of 380 to 500 nm in a spectral reflectance curve)) was determined in the chromaticity diagram from the average a* value and the b* value of the region.

The inks were applied to a poly(lactic acid) sheet to have the color tone of the identified complementary color. The pattern was the same as the pattern (circular) of the region corresponding to the freckle in the freckle model. The printed sheet was dried at 40°C under reduced pressure (-0.5 kPa) for 60 minutes to form a complementary color layer on the poly(lactic acid) sheet. The complementary color layer had a thickness of 800 nm.

The white ink was then applied to the complementary color layer. The print pattern was almost the same as the shape of the complementary color layer. The printed sheet was dried at 40°C under reduced pressure (-0.5 kPa) for 60 minutes to form a light scattering layer on the complementary color layer. The light scattering layer was covered with another identical light scattering layer. The light scattering layer had a total thickness of 1600 nm.

The inks were then applied to the light scattering layer to form a skin-colored coloring layer. The pattern of the coloring layer was a circular pattern concentric with the complementary color layer and the light scattering layer and had a diameter slightly larger than the complementary color layer and the light scattering layer. The printed sheet was dried at 40°C under reduced pressure (-0.5 kPa) for 60 minutes to form a coloring layer on the light scattering layer. The coloring layer was covered with another identical coloring layer. The coloring layer had a total thickness of 1600 nm.

### 2-2. Comparative Example

Eight layers of the same light scattering layer and two layers of the same coloring layer as in the example were formed on the same substrate as in the example to produce a cosmetic sheet.

### 3. Evaluation

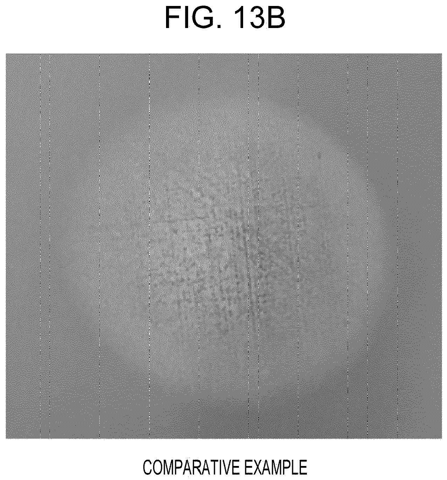

Fig. 13A is an image of the cosmetic sheet produced in the present example put over the freckle model. Fig. 13B is an image of the cosmetic sheet produced by a conventional technique in the comparative example put over the freckle model. As is clear from Fig. 13A, the cosmetic sheet according to the present example obscured the freckle irrespective of its smaller number of layers.

### Industrial Applicability

A cosmetic sheet according to the present disclosure put on the skin can sufficiently obscure a discolored region and is less likely to arouse a feeling of thickness. Thus, a cosmetic sheet according to the present disclosure is useful for various types of skin coloring and beautification.

### Reference Signs List

100 makeup support system 200 image-processing apparatus 201 illumination unit 202 camera 203 display screen 220 image-capturing unit 230 image analyzer 240 sheet information providing unit 250 finish checker 260 print controller 270 image data storage 280 information storage 290 complementary color layer information table 300 printer 520 thin film 521 complementary color layer 522 light scattering layer 523 coloring layer

## Claims

1. A cosmetic sheet to be put on a discolored portion of skin, comprising:
a thin film, one surface of which is to be put on the skin;
a complementary color layer formed by applying an ink with a color tone complementary to the color of the discolored portion to the other surface of the thin film;
a light scattering layer formed by applying an ink containing a reflective material to the complementary color layer and;
a coloring layer placed formed by applying a coloring material ink to the light scattering layer, wherein each of the inks for the complementary color layer, the light scattering layer, and the coloring layer further contains a higher alcohol having three to five carbon atoms, purified water, and an acrylic resin,
the acrylic resin contains at least one polymer selected from the group consisting of homopolymers of an acrylic monomer and copolymers of two or more acrylic monomers, and
wherein the complementary color layer has a basic color of blue and has a maximum at a wavelength in the range of 380 to 570 nm in its spectral reflectance curve.

2. The cosmetic sheet according to Claim 1, wherein the light scattering layer has a thickness in the range of 0.1 to 10 when the complementary color layer has a thickness of 1.

3. The cosmetic sheet according to Claim 1, wherein the complementary color layer also has skin color.

4. The cosmetic sheet according to Claim 3, wherein the complementary color layer has a color tone of peach orange, purple, green, or pink.

5. The cosmetic sheet according to Claim 1, wherein the complementary color layer has different shades of color in a thickness direction.

6. A method for producing a cosmetic sheet to be put on a discolored portion of skin, the method comprising:
a step of applying a complementary color layer ink containing a coloring material complementary to the color of the discolored portion to one surface of a thin film, the other surface of which is to be put on the skin, and drying the complementary color layer ink to form a complementary color layer;
a step of applying a light scattering layer ink containing a reflective material to the complementary color layer and drying the light scattering layer ink to form a light scattering layer; and
a step of applying a coloring layer ink containing a coloring material to the light scattering layer and drying the coloring layer ink to form a coloring layer,
wherein each of the inks for the complementary color layer, the light scattering layer, and the coloring layer further contains a higher alcohol having three to five carbon atoms, purified water, and an acrylic resin, and
the acrylic resin contains at least one polymer selected from the group consisting of homopolymers of an acrylic monomer and copolymers of two or more acrylic monomers.

7. The method for producing a cosmetic sheet according to Claim 7, wherein the light scattering layer has a thickness in the range of 0.1 to 10 when the complementary color layer has a thickness of 1.

## Patentansprüche

1. Kosmetisches Blatt zum Auflegen auf einen verfärbten Hautabschnitt, umfassend:
eine dünne Folie, deren eine Fläche auf die Haut aufgelegt wird;
eine Schicht in Komplementärfarbe, die ausgebildet wird, indem eine Druckfarbe mit einem Farbton, der komplementär zu der Farbe des verfärbten Abschnitts ist, auf die andere Fläche der dünnen Folie aufgebracht wird;
eine lichtstreuende Schicht, die durch Aufbringen einer Druckfarbe, die ein reflektierendes Material enthält, auf die Schicht in Komplementärfarbe ausgebildet wird, und;
eine färbende Schicht, die durch Aufbringen einer Druckfarbe aus färbendem Material auf die lichtstreuende Schicht ausgebildet wird,
wobei jede der Druckfarben für die Schicht in Komplementärfarbe, die lichtstreuende Schicht und die färbende Schicht des Weiteren einen höheren Alkohol mit drei bis fünf Kohlenstoffatomen, gereinigtes Wasser sowie ein Acrylharz enthält,
das Acrylharz wenigstens ein Polymer enthält, das aus der Gruppe ausgewählt wird, die aus Homopolymeren eines Acrylmonomers und Copolymeren von zwei oder mehr Acrylmonomeren besteht, und
wobei die Schicht in Komplementärfarbe als eine Grundfarbe Blau aufweist und in der Kurve ihres spektralen Reflexionsgrades ein Maximum bei einer Wellenlänge im Bereich von 380 bis 570 nm hat.

2. Kosmetisches Blatt nach Anspruch 1, wobei die lichtstreuende Schicht eine Dicke im Bereich von 0,1 bis 10 hat, wenn die Schicht in Komplementärfarbe eine Dicke von 1 hat.

3. Kosmetisches Blatt nach Anspruch 1, wobei die Schicht in Komplementärfarbe ebenfalls Hautfarbe hat.

4. Kosmetisches Blatt nach Anspruch 3, wobei die Schicht in Komplementärfarbe einen Farbton von Pfirsich-Orange, Violett, Grün oder Pink aufweist.

5. Kosmetisches Blatt nach Anspruch 1, wobei die Schicht in Komplementärfarbe unterschiedliche Farbschattierungen in einer Dickenrichtung aufweist.

6. Verfahren zum Herstellen eines kosmetischen Blattes, das auf einen verfärbten Hautabschnitt aufgelegt wird, wobei das Verfahren umfasst:
einen Schritt des Aufbringens einer Druckfarbe für eine Schicht in Komplementärfarbe, die ein färbendes Material enthält, das komplementär zu der Farbe des verfärbten Abschnitts ist, auf eine Oberfläche einer dünnen Folie, deren andere Fläche auf die Haut aufgelegt wird, und des Trocknens der Druckfarbe für eine Schicht in Komplementärfarbe zum Ausbilden einer Schicht in Komplementärfarbe;
einen Schritt des Aufbringens einer Druckfarbe für eine lichtstreuende Schicht, die ein reflektierendes Material enthält, auf die Schicht in Komplementärfarbe und des Trocknens der Druckfarbe für eine lichtstreuende Schicht zum Ausbilden einer lichtstreuenden Schicht; sowie
einen Schritt des Aufbringens einer Druckfarbe für eine färbende Schicht, die ein färbendes Material enthält, auf die lichtstreuende Schicht und des Trocknens der Druckfarbe für eine färbende Schicht zum Ausbilden einer färbenden Schicht,
wobei jede der Druckfarben für die Schicht in Komplementärfarbe, die lichtstreuende Schicht und die färbende Schicht des Weiteren einen höheren Alkohol mit drei bis fünf Kohlenstoffatomen, gereinigtes Wasser sowie ein Acrylharz enthält, und
das Acrylharz wenigstens ein Polymer enthält, das aus der Gruppe ausgewählt wird, die aus Homopolymeren eines Acrylmonomers und Copolymeren von zwei oder mehr Acrylmonomeren besteht.

7. Verfahren zum Herstellen eines kosmetischen Blattes nach Anspruch 7, wobei die lichtstreuende Schicht eine Dicke im Bereich von 0,1 bis 10 hat, wenn die Schicht in Komplementärfarbe eine Dicke von 1 hat.

## Revendications

1. Feuille cosmétique à placer sur une portion décolorée de la peau, comprenant :
un film fin, dont une surface doit être placée sur la peau ;
une couche de couleur complémentaire formée en appliquant une encre ayant un ton de couleur complémentaire de la couleur de la portion décolorée à l'autre surface du film fin ;
une couche de diffusion de lumière formée en appliquant une encre contenant un matériau de réflexion à la couche de couleur complémentaire et ;
une couche colorante placée formée en appliquant une encre de matériau colorant à la couche de diffusion de lumière,
chacune des encres pour la couche de couleur complémentaire, la couche de diffusion de lumière, et la couche colorante contenant en outre un alcool supérieur ayant trois à cinq atomes de carbone, de l'eau purifiée, et une résine acrylique,
la résine acrylique contenant au moins un polymère sélectionné dans le groupe constitué des homopolymères d'un monomère acrylique et des copolymères de deux ou plusieurs monomères acryliques, et
la couche de couleur complémentaire ayant une couleur de base de bleu et ayant un maximum à une longueur d'onde située dans la plage de 380 à 570 nm dans sa courbe de réflectance spectrale.

2. Feuille cosmétique selon la revendication 1, la couche de diffusion de lumière ayant une épaisseur située dans la plage de 0, 1 à 10 lorsque la couche de couleur complémentaire présente une épaisseur de 1.

3. Feuille cosmétique selon la revendication 1, la couche de couleur complémentaire ayant également la couleur de la peau.

4. Feuille cosmétique selon la revendication 3, la couche de couleur complémentaire ayant un ton de couleur d'orange pêche, de violet, de vert, ou de rose.

5. Feuille cosmétique selon la revendication 1, la couche de couleur complémentaire ayant différentes nuances de couleur dans un sens de l'épaisseur.

6. Procédé de production d'une feuille cosmétique à placer sur une portion décolorée de la peau, le procédé comprenant :
une étape d'application d'une encre de couche de couleur complémentaire contenant un matériau colorant complémentaire de la couleur de la portion décolorée à une surface d'un film fin, dont l'autre surface doit être placée sur la peau, et le séchage de l'encre de couche de couleur complémentaire pour former une couche de couleur complémentaire ;
une étape d'application d'une encre de couche de diffusion de lumière contenant un matériau de réflexion à la couche de couleur complémentaire et le séchage de l'encre de couche de diffusion de lumière pour former une couche de diffusion de lumière ; et
une étape d'application d'une encre de couche colorante contenant un matériau colorant à la couche de diffusion de lumière et le séchage de l'encre de couche colorante pour former une couche colorante,
chacune des encres pour la couche de couleur complémentaire, la couche de diffusion de lumière, et la couche colorante contenant en outre un alcool supérieur ayant trois à cinq atomes de carbone, de l'eau purifiée, et une résine acrylique, et
la résine acrylique contenant au moins un polymère sélectionné dans le groupe constitué des homopolymères d'un monomère acrylique et des copolymères de deux ou plusieurs monomères acryliques.

7. Procédé de production d'une feuille cosmétique selon la revendication 7, la couche de diffusion de lumière ayant une épaisseur située dans la plage de 0,1 à 10 lorsque la couche de couleur complémentaire présente une épaisseur de 1.
